# EUROPEAN PATENT APPLICATION

(11) **EP 0 618 312 A1**
(43) Date of publication of application: **05.10.1994**
(21) Application number: 94870038.0
(22) Date of filing: 01.03.1994
(51) Int. Cl.: C25B 3/04, C07D 333/36

(54) **Process for obtaining n-acetyl homocysteine thiolactone from DL-homocystine by electrochemical methods**

(30) Priority: 02.03.1993 ES 9300403
(71) Applicant: PRODESFARMA, S.A., E-08960 Sant Just Desvern (Barcelona) (ES)
(72) Inventor: Garcia Garcia, Vicente, Torrevieja, Alicante (ES); Sanchez Cano, Gaspar, Alicante (ES); Montiel Leguay, Vicente, Santa Pola (Alicante) (ES); Elias Vilarelle, Eduardo, Barcelona (ES); Ginebreda Martin, Antonio, Barcelona (ES); Aldaz Riera, Antonio, Campello (Alicante) (ES)
(74) Representative: Schmitz, Yvon

(57) **Abstract**

A process for obtaining N-acetyl homocysteine thiolactone of the formula (I) from DL-homocystine by electrochemical methods. An aqueous solution of DL-homocystine and a mineral acid are submitted to electrolysis in an electrolytic cell with the catholyte and anolyte separated. The catholyte consists of the above solution and the anolyte can be any saline solution. The electrodes can be flat, or three-dimensional, forming a compact or fluidized bed. The current tension is between 10 mA/cm² and 2 A/cm²; the temperature is comprised between 0 and 90°C . The solution obtained in the catholyte is concentrated, dried and acetylated to obtain the compound (I), with a high degree of purity.

The compound of the invention can be used for the therapy of liver complaints and as a mucolytic.

## Description

The invention refers to a general process for obtaining N-acetyl homocysteine thiolactone (cythiolone) of the formula (I).

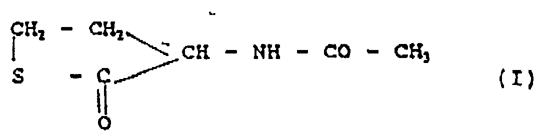

In the chemical literature we find processes for obtaining this product industrially that are protected by patents.

Some methods are based on reaction with sodium and liquid ammonia from N-acetyl methionine. 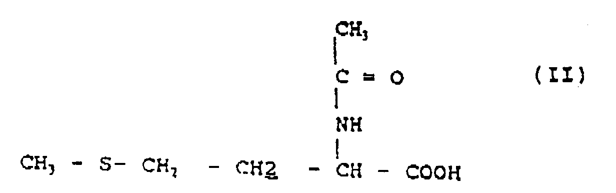
(German patent DE 1.134.683)
or from methionine 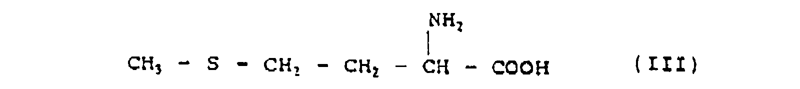
(Japanese patent JP 1.376) to eliminate the methyl group and later cycling, or acetylation and cycling, to obtain cythiolone.

These processes use an expensive and dangerous technology, as they involve the handling of liquid ammonia under pressure and/or at low temperature (-50°C), and the addition of metal sodium with the drawbacks and dangers that this involves.

By treating the methionine in sulfuric acid we obtain DL-homocystine.

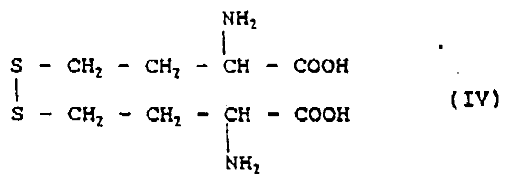

This can be reduced with tin and hydrochloric acid (Journal of Biological Chemisty, 112, p. 149, 1935-36) or with zinc in acetic acid (Japanese patent JP 3.420) to give homocysteine thiolactone 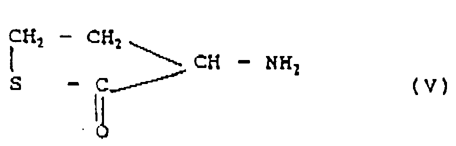
which when acetylated according to usual methods gives cythiolone.

These processes involve the use of metals in acid medium, in which hydrogen is produced. These reactions are dangerous as this release of hydrogen is difficult to control, and they leave metal residues that are difficult to eliminate or recycle whereby the environment is damaged.

Different methods of acetylation of homocysteine thiolactone are described in several patents (Japanese patent JP 16.712 and French patents FR 2.147.468 and 2.219.877).

The process according to the invention is characterized fundamentally in that it makes the DL-honiacystine react 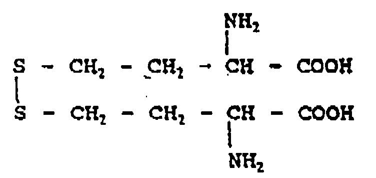
electrochemically in acid medium in order to obtain a salt of homocystine thiolactone 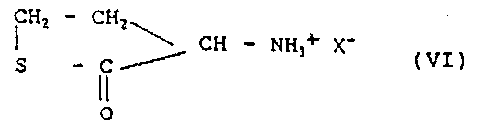
which when acetylated by suitable methods gives cythiolone.

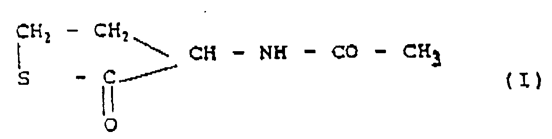

The process that is the object of the present invention is particularly suitable for industrial application whereby it should be noted in comparison with prior art that it is an easily controllable method and that it does not leave residues that deteriorate the environment, as the raw material used is the electric current.

### DESCRIPTION OF THE METHOD

Homocysteine thiolactone is obtained electrochemically by electroreduction in a medium composed of acid water of DL-homocystine.

The system of electrosynthesis is formed by one or several cathodes which can be made of : graphite, tin, zinc, lead, mercury, copper, titanium, platinum, platinized titanium or any steel or an alloy containing iron, and by one or several anodes made of lead, graphite, dimensionally stable anodes, titanium, platinized titanium or lead titanium.

The solution that is in contact with the cathode (called the catholyte) and which is in contact with the anode (called the anolyte) are separated either by a membrane that is selective to cations or anions, or by any other type of separator.

The assembly of the electrosynthesis cell is composed of a variable number of electrodes, according to the production needs, which act either as a cathode, or as an anode.

The electrodes may be flat or of any other form or structure, whether or not arranged in parallel, in accordance with a typical filter press arrangement; they may form a compact or fluidized bed, and lastly the electrode may be porous, operating as such in its three dimensions (three-dimensional electrodes).

The connection of electrodes to the source can be made according to a single-pole, two-pole or mixed assembly.

The catholyte is composed of an aqueous solution of DL-homocystine and a mineral acid.

The anolyte is composed of an aqueous solution of any saline electrolyte.

The temperature at which the electrolysis is performed will be comprised between 0 and 90°C.

The current density will be chosen between 10 mA/cm² and 2 A/cm², and does not necessarily have to remain constant throughout the electrolysis.

A sufficient amount of charge is circulated to reduce all the initial DL-homocystine that is in the catholyte. This marks the end of the electrochemical reaction, and the product is then isolated.

To do this, the catholyte is concentrated in a vacuum at 40°C until a paste is obtained; the minimum amount of ethanol needed is added and the solid is filtered. After drying in a vacuum at 45°C, this gives us homocysteine thiolactone with a very high degree of purity ( 99%) and with a material yield of 80 to 95 % according to the specific conditions of electrolysis.

This product is acetylated by known methods, giving N-acetyl homocysteine thiolactone with a very high degree of purity and with an overall yield of 75 to 90%.

Below we give an example of specific reaction conditions, whereby this must not be considered as a limitation of the object of the invention.

### EXAMPLE

In a filter press electrochemical reactor, with a cathode and an anode having an area of 0.15 m², a catholyte (30 litres) with a composition of 0.25 molar DL- homocystine and 1 molar hydrochloric acid, and an anolyte (30 litres) with a composition of 1 molar sulfuric acid are circulated. The cathode is a graphite plate of 0,5 cm thickness and the anode is a dimensionally stable electrode (DSA) with a low oxygen overvoltage.

In order to separate the anolyte from the catholyte, a membrane is used at 40mA/cm² throughout the whole reaction, at a temperature of 20 to 25°C. The electrolysis ended when all the initial DL-homocystine was consumed (20 hours).

The catholyte was then concentrated in a vacuum at 40°C and 500 ml of ethanol were added and the insoluble solid was filtered, which after drying in a vacuum at 45°C, gave 2.31 kg of pure homocysteine thiolactone monohydrochloride. The yield was of 91%. Without later purification, this solid is treated with 11 l of toluene at 90°C and 2.3 kg of acetic anhydride are added drop by drop. The solvent is distilled to eliminate the acetic acid that has formed and it is crystallized with 10 l of toluene, giving 2.1 kg of cythiolone of p.f. 111-112°C. The yield is of 94% compared with homocysteine thiolactone.

## Claims

1. Process for obtaining N-acetyl homocysteine thiolactone of the formula 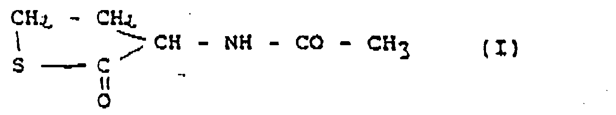 from DL-homocystine by electrochemical methods, characterized in that in an electrolytic cell, with separation between the catholyte and the anolyte, an aqueous solution of DL-homocystine and a mineral acid that forms the catholyte, and an aqueous solution of any salt that forms the anolyte, are submitted to electrolysis in suitable conditions of current tension and temperature and with flat, three-dimensional electrodes, an aqueous solution of DL-homocysteine thiolactone being obtained, which can be isolated as a salt of the corresponding acid or concentrated and acetylated to give N-acetyl homocysteine thiolactone with a very high degree of purity.

2. Process according to claim 1, characterized in that the electrodes used may be flat or of any geometric shape and three-dimensional, and they form a compact bed or fluidized bed.

3. Process according to claim 2, characterized in that the number of electrodes for each electrolytic cell is variable whereby it is possible to work with a single-pole, two-pole or mixed assembly.

4. Process according to claim 2, characterized in that the cathode may be made of graphite, tin, zinc, lead, mercury, copper, titanium, platinum, platinized titanium or any other steel or alloy containing iron.

5. Process according to claim 2, characterized in that the anode may be made of lead, graphite, dimensionally stable anodes, tianium, platinum, platinized titanium or lead titanium.

6. Process according to claim 1, characterized in that in the electrolytic cell, the catholyte and the anolyte are separated by a membrane that is selective to cations or anions, or by any separator, whether or not it is selective.

7. Process according to claim 1, characterized in that the catholyte is made up of an aqueous solution of DL-homocystine and a mineral acid, and the anolyte is made up of an aqueous solution of any salt.

8. Process according to claim 1, characterized in that the working temperature is comprised between 0 and 90°C.

9. Process according to claims 1 and 8, characterized in that the working temperature is more specifically comprised between 20 and 40°C.

10. Process according to claim 1, characterized in that the working current density is comprised between 10 mA/cm² and 2 A/cm², whereby it does not necessarily have to remain fixed throughout the electrolysis.

11. Process according to claims 1 and 10, characterized in that the specific density of the working current is comprised between 35 and 400 mA/cm².

12. Process accroding to claim 1, characterized in that the DL-homocysteine thiolactone can be isolated from the solution obtained in the catholyte as a salt of the corresponding acid 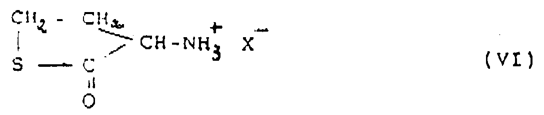 where X = Cl⁻, Br⁻, 1/2SO₄²⁻, 1/3 PO₄³⁻, ... .

13. Process according to claim 1, characterized in that the DL-homocysteine thiolactone (V) can be separated by concentrating the catholyte in a vacuum, precipitating it with an organic solvent, filtering the solid obtained and drying it in a vacuum.

14. Process according to claims 1 and 13, characterized in that the DL-homocysteine thiolactone obtained is acetylated to give N-acetyl homocysteine thiolactone, with a very high degree of purity.
